# EUROPEAN PATENT APPLICATION

(11) **EP 4 386 771 A1**
(43) Date of publication of application: **19.06.2024**
(21) Application number: 22875407.3
(22) Date of filing: 23.03.2022
(51) Int. Cl.: G16H 20/00, G16H 40/00

(54) **MEDICAL INFORMATION PROCESSING SYSTEM, DETERMINATION METHOD, AND PROGRAM**

(30) Priority: 28.09.2021 JP 2021158232
(71) Applicant: Sony Group Corporation, Tokyo 108-0075 (JP)
(72) Inventor: TAKEMOTO Masaya, Tokyo 108-0075 (JP)
(74) Representative: D Young & Co LLP
(86) International application number: PCT/JP2022/013556
(87) International publication number: WO 2023/053524

(57) **Abstract**

The present disclosure relates to a medical information processing system, a determining method, and a program capable of avoiding an inappropriate use of medical device software.

A list management unit manages a software list in which contraindication information is set for each registered software, an information acquisition unit acquires patient information regarding a patient to whom requested software requested to be activated is used and device information regarding an electronic device used for the patient, and a contraindication determination unit determines whether or not activation of the requested software should be contraindicated according to whether or not the patient information or the device information corresponds to the contraindication information set in the requested software in the software list. The present disclosure is applicable to an operating room system.

## Description

### TECHNICAL FIELD

The present disclosure relates to a medical information processing system, a determining method, and a program, and especially relates to a medical information processing system, a determining method, and a program capable of preventing an inappropriate use of medical device software.

### BACKGROUND ART

In an operating room, a double check is performed in many occasions, such as a confirmation of a name of an operator who enters the room and of a drug to be administered to a patient, and a confirmation of a surgical method and an operation site at the start of an operation, to prevent a serious mistake.

A medical practice that is not adapted to a particular disease and prohibited due to an adverse effect is referred to as a contraindication. Many confirmations described above are also performed to prevent contraindications.

For example, Patent Document 1 discloses detecting a drug or a tool used in an endoscopic examination by image processing and determining whether or not the detected drug or tool violates contraindication information of a patient.

### CITATION LIST

### PATENT DOCUMENT

Patent Document 1: Japanese Patent Application Laid-Open No. 2018-92673

### SUMMARY OF THE INVENTION

### PROBLEMS TO BE SOLVED BY THE INVENTION

With recent spread of medical device software, it is assumed that specific software is contraindicated for a specific disease in the future. It is desirable that such contraindication regarding the software be checked by a system.

The present disclosure has been made in view of such circumstances, and this can prevent inappropriate use of the medical device software.

### SOLUTIONS TO PROBLEMS

A medical information processing system according to the present disclosure is a medical information processing system including a list management unit that manages a software list in which contraindication information is set for each registered software, an information acquisition unit that acquires patient information regarding a patient to whom requested software requested to be activated is used and device information regarding an electronic device used for the patient, and a contraindication determination unit that determines whether or not activation of the requested software should be contraindicated according to whether or not the patient information or the device information corresponds to the contraindication information set in the requested software in the software list.

A determining method according to the present disclosure is a determining method including, by a medical information processing system, managing a software list in which contraindication information is set for each registered software, acquiring patient information regarding a patient to whom requested software requested to be activated is used and device information regarding an electronic device used for the patient, and determining whether or not activation of the requested software should be contraindicated according to whether or not the patient information or the device information corresponds to the contraindication information set in the requested software in the software list.

A program according to the present disclosure is a program of a computer of managing a software list in which contraindication information is set for each registered software, acquiring patient information regarding a patient to whom requested software requested to be activated is used and device information regarding an electronic device used for the patient, and determining whether or not activation of the requested software should be contraindicated according to whether or not the patient information or the device information corresponds to the contraindication information set in the requested software in the software list.

In the present disclosure, a software list in which contraindication information is set for each registered software is managed, patient information regarding a patient to whom requested software requested to be activated is used and device information regarding an electronic device used for the patient are acquired, and it is determined whether or not activation of the requested software should be contraindicated according to whether or not the patient information or the device information corresponds to the contraindication information set in the requested software in the software list.

### BRIEF DESCRIPTION OF DRAWINGS

Fig. 1 is a diagram illustrating an outline of an operating room system to which the technology according to the present disclosure can be applied.
Fig. 2 is a block diagram illustrating a functional configuration example of a medical information processing system.
Fig. 3 is a flowchart for illustrating a flow of software activation determination processing.
Fig. 4 is a diagram illustrating an example of a software list.
Fig. 5 is a block diagram illustrating another functional configuration example of the medical information processing system.
Fig. 6 is a flowchart for illustrating a flow of software activation determination processing.
Fig. 7 is a diagram illustrating an example of a warning screen.
Fig. 8 is a flowchart for illustrating a flow of software activation determination processing.
Fig. 9 is a diagram illustrating another example of the software list.
Fig. 10 is a block diagram illustrating still another functional configuration example of the medical information processing system.
Fig. 11 is a flowchart for illustrating a flow of list update processing.
Fig. 12 is a flowchart for illustrating a flow of software activation determination processing.
Fig. 13 is a block diagram illustrating still another functional configuration example of the medical information processing system.
Fig. 14 is a flowchart for illustrating a flow of software activation determination processing.
Fig. 15 is a flowchart for illustrating a flow of software activation determination processing.
Fig. 16 is a diagram illustrating a configuration example of a computer.

### MODE FOR CARRYING OUT THE INVENTION

A mode for carrying out the present disclosure (hereinafter, referred to as an embodiment) will be hereinafter described. Note that, the description is given in the following order.

1. Background
2. Outline of Operating Room System
3. First Embodiment (Software Activation Determination Based on Contraindication Information)
4. Second Embodiment (Software Activation Determination Including Warning Display)
5. Third Embodiment (Software Activation Determination in Emergency)
6. Fourth Embodiment (Update of Software List and Software Activation Determination)
7. Fifth Embodiment (Software Activation Determination Based on Update of Contraindication Information and Patient Information)
8. Sixth Embodiment (Software Activation Determination Based on Contraindication Information with Condition)
9. Configuration Example of Computer

### <1. Background>

In an operating room, a double check is performed in many occasions, such as a confirmation of a name of an operator who enters the room and of a drug to be administered to a patient, and a confirmation of a surgical method and an operation site at the start of an operation, to prevent a serious mistake.

A medical practice that is not adapted to a particular disease and prohibited due to an adverse effect is referred to as a contraindication. Many confirmations described above are also performed to prevent contraindications.

However, even in recent years in which the confirmation such as the double check by doctors and nurses is conventionalized, there are many cases where a patient is seriously damaged by implementation of contraindications due to human errors.

With recent spread of medical device software, it is assumed that specific software is contraindicated for a specific disease in the future. That is, use of the medical device software itself is assumed to be the contraindications for the patient.

It is desirable that such contraindication regarding the software be checked by a system in order to eliminate human errors.

Then, in the technology according to the present disclosure, it is determined whether or not activation of the medical device software should be prohibited due to a possible adverse effect by a patient-specific factor, and prevention of inappropriate use of the medical device software is implemented by controlling the activation of the software.

### <2. Outline of Operating Room System>

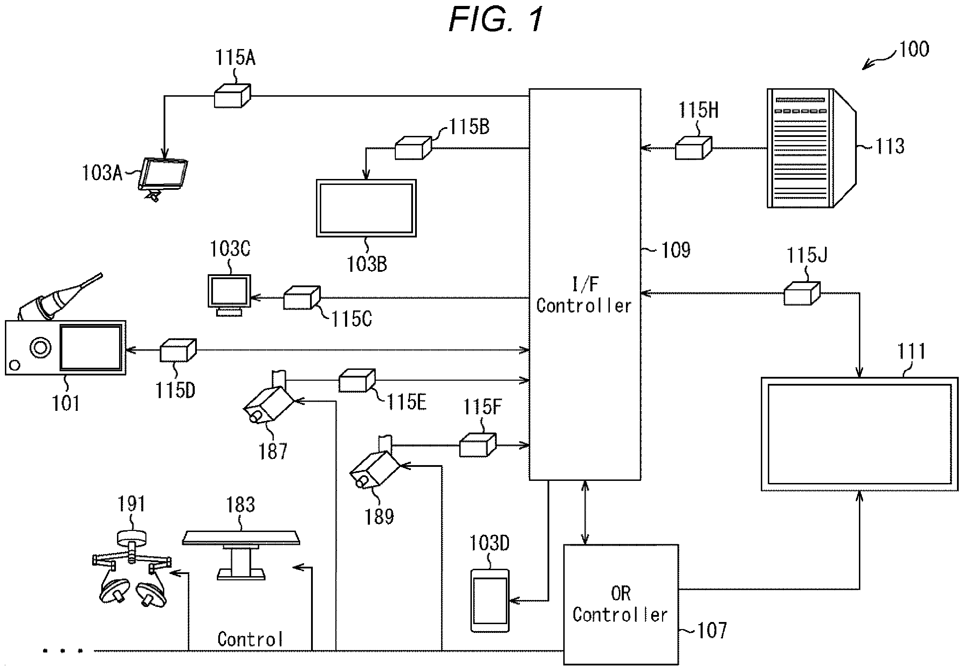

Fig. 1 is a diagram illustrating an outline of an operating room system 100 to which the technology according to the present disclosure can be applied.

In the operating room system 100 illustrated in Fig. 1, a group of devices installed in an operating room is connected so as to be able to cooperate with one another via an operating room controller (OR controller) 107 and an input/output controller (I/F controller) 109. The operating room system 100 includes an Internet protocol (IP) network capable of transmitting and receiving 4K/8K images, and input and output images and control information directed to each device are transmitted and received via the IP network.

Various devices might be installed in the operating room.

In an example in Fig. 1, a device group 101 including various devices used for endoscopic surgery, a ceiling camera 187 that captures an image of hands of an operator, a surgical field camera 189 that captures an image of a state of an entire operating room, a plurality of display devices 103A to 103D, a patient bed 183, and a light 191 are illustrated. Both the ceiling camera 187 and the surgical field camera 189 are installed on the ceiling of the operating room. In addition to the illustrated endoscope, various medical devices that acquire images, such as a master-slave endoscopic surgical robot and an X-ray imaging device, may be applied to the device group 101.

The display devices 103A to 103C, the device group 101, the ceiling camera 187, and the surgical field camera 189 are connected to the input/output controller 109 via IP converters 115A to 115F, respectively. Hereinafter, in a case where the IP converters 115A to 115F and the like are not especially distinguished from one another, they are simply referred to as the IP converter 115.

Each of the IP converters 115D, 115E, and 115F on an input source side (camera side) that inputs an image performs IP conversion on an image from each medical imaging device (endoscope, surgical microscope, X-ray imaging device, surgical field camera, pathological imaging device and the like) and transmits the converted image over a network. Each of the IP converters 115A to 115C on an image output side (monitor side) that outputs an image converts the image transmitted via the network into a format specific to a monitor and outputs the converted image. The IP converter 115 on the input source side functions as an encoder, and the IP converter 115 on the image output side functions as a decoder. The input source includes, for example, a video source.

The IP converter 115 can have various image processing functions. For example, the IP converter 115 can have a function of executing resolution conversion processing depending on a destination, rotation correction and camera shake correction on an endoscopic image, object recognition processing, partial processing such as characteristic information extraction for analysis by a server to be described later and the like.

These image processing functions may be specific to the connected medical image devices, or may be externally upgradable. The IP converter 115 on the image output side (monitor side) can also perform processing such as composition of a plurality of images (PinP processing and the like) and superimposition of annotation information.

A protocol conversion function of the IP converter 115 is a function of converting a received signal into a converted signal that conforms to a communication protocol that may be communicated on a network such as the Internet, for example. Any communication protocol may be set as the communication protocol. Furthermore, a protocol-convertible signal received by the IP converter 115 is a digital signal such as an image signal or a pixel signal, for example. The IP converter 115 may be incorporated in a device on the input source side or a device on the image output side.

The device group 101 belongs to, for example, an endoscopic surgery system, and includes an endoscope, a display device that displays an image captured by the endoscope and the like. In contrast, the display devices 103A to 103D, the patient bed 183, and the light 191 are devices provided in the operating room separately from the endoscopic surgery system. Such devices used for operation or diagnosis are also referred to as medical devices. The operating room controller 107 and/or the input/output controller 109 controls operations of the medical devices in cooperation. Furthermore, in a case where a medical image acquisition device such as a surgical robot (surgical master-slave) system and an X-ray imaging device is included in the operating room, such devices are also connectable as the device group 101.

The operating room controller 107 comprehensively controls processing regarding image display in the medical devices.

Here, out of the devices included in the operating room system 100, the device group 101, the ceiling camera 187, and the surgical field camera 189 may be devices that have a function of transmitting information to be displayed during operation (hereinafter, also referred to as display information) (hereinafter, also referred to as source devices). Furthermore, the display devices 103A to 103D may be devices that output the display information (hereinafter, also referred to as destination devices).

The operating room controller 107 has a function of controlling the operation of the source device and the destination device, acquiring the display information from the source device, transmitting the display information to the destination device, and allowing the destination device to display or record the display information. The display information includes various images captured during operation, various pieces of information regarding the operation (for example, physical information of a patient, and information regarding a past examination result and a surgical method) and the like.

Specifically, information regarding an image of a surgical site in a body cavity of the patient captured by the endoscope can be transmitted as the display information from the device group 101 to the operating room controller 107. Furthermore, information regarding the image of the hands of the operator captured by the ceiling camera 187 may be transmitted to the operating room controller 107 as the display information from the ceiling camera 187. Furthermore, information regarding the image of the state of the entire operating room captured by the surgical field camera 189 may be transmitted to the operating room controller 107 as the display information from the surgical field camera 189. In a case where the operating room system 100 includes other devices having an imaging function, the operating room controller 107 may acquire information regarding images captured by the other devices as the display information from the other devices.

The operating room controller 107 allows at least one of the display devices 103A to 103D serving as the destination devices to display the acquired display information (that is, the images captured during the operation and the various pieces of information regarding the operation). In the example in Fig. 1, the display device 103A is a display device installed on the ceiling of the operating room in a suspended manner, and the display device 103B is a display device installed on a wall surface of the operating room. The display device 103C is a display device installed on a desk in the operating room, and the display device 103D is a mobile device (for example, a tablet personal computer (PC) or a smartphone) having a display function.

The input/output controller 109 is configured as one of input/output controllers that control input/output of the image signal to/from a connected device. For example, the input/output controller 109 controls input/output of the image signal on the basis of control of the operating room controller 107. The input/output controller 109 includes, for example, an IP switcher and the like, and controls high-speed transfer of the image signal between devices arranged on the IP network.

The operating room system 100 may include a device located outside the operating room. The device located outside the operating room may be, for example, a server connected to a network constructed inside or outside a hospital, a PC used by a medical staff, a projector installed in a conference room of the hospital and the like. In a case where such external device is present outside the hospital, the operating room controller 107 can also allow a display device of another hospital to display the display information via a teleconference system and the like for telemedicine.

An external server 113 is, for example, an in-hospital server or a cloud server located outside the operating room, and may be used for image analysis, data analysis and the like. In this case, the information of the image in the operating room may be transmitted to the external server 113, and additional information may be generated by big data analysis or recognition/analysis processing using AI (machine learning) by the external server 113 to be fed back to the display device in the operating room. At that time, an IP converter 115H connected to the input/output controller 109 in the operating room transmits data to the external server 113 for image analysis. The data to be transmitted may be a surgical image itself captured by the endoscope and the like, metadata extracted from the image, data indicating an operation status of a connected device and the like.

The operating room system 100 is provided with a centralized operation panel 111. Via the centralized operation panel 111, a user can give the operating room controller 107 an instruction regarding input/output control of the input/output controller 109 and an instruction regarding an operation of the connected devices. Furthermore, the user can switch the image displays via the centralized operation panel 111. The centralized operation panel 111 is configured as a touch panel provided on a display surface of the display device. The centralized operation panel 111 is connected to the input/output controller 109 via an IP converter 115J.

In the operating room system 100, the IP network may be configured as a wired network, or a part or all of the IP network may be configured as a wireless network. For example, the IP converter 115 on the input source side having a wireless communication function may transmit a received image to the IP converter 115 on the image output side via a wireless communication network such as a fifth generation mobile communication system (5G) or a sixth generation mobile communication system (6G) .

A functional configuration example of the operating room system 100 according to the embodiment of the present disclosure will be hereinafter described.

### <3. First Embodiment>

### (Functional Configuration Example of Operating Room System)

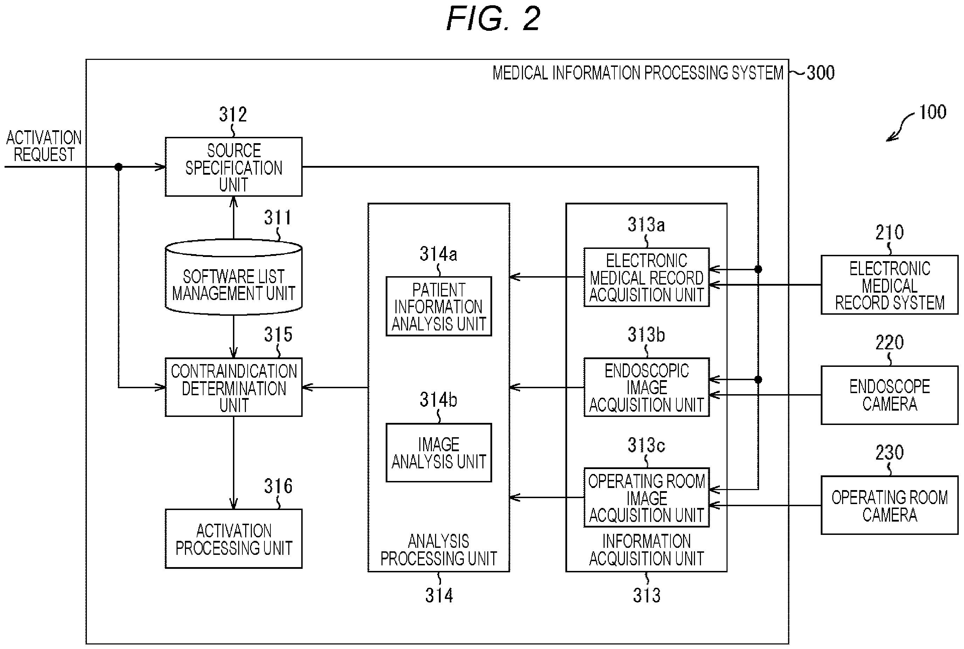

Fig. 2 is a diagram illustrating a functional configuration example of an operating room system 100 according to a first embodiment of the present disclosure.

The operating room system 100 in Fig. 2 includes an electronic medical record system 210, an endoscope camera 220, an operating room camera 230, and a medical information processing system 300.

The electronic medical record system 210, the endoscope camera 220, and the operating room camera 230 are examples of an electronic device configured as an information management system that inputs information regarding a patient (patient information), and an input source that inputs the above-described image and information regarding each electronic device (device information), for example. Hereinafter, these electronic devices are also collectively referred to as a source.

The electronic medical record system 210 manages an electronic medical record, which is clinical information for each patient, and inputs the same to the medical information processing system 300.

The endoscope camera 220 is one of the medical imaging devices included in the above-described device group 101 forming the endoscopic surgery system, and inputs a captured endoscopic image to the medical information processing system 300.

The operating room camera 230 is one of imaging devices that captures an image of the inside of the operating room, such as the ceiling camera 187 and the surgical field camera 189 described above, and inputs the captured operating room image to the medical information processing system 300.

The medical information processing system 300 is configured as a medical platform system capable of executing a plurality of pieces of installed medical device software. The medical information processing system 300 controls activation of the medical device software on the basis of data such as the electronic medical record and image acquired from the above-described source in response to an activation request of the medical device software from a surgical staff such as a doctor or a nurse who is a user. Hereinafter, patient information such as the electronic medical record and images and device information acquired from the source are also referred to as acquired information.

The medical device software herein mentioned is application software executed for implementing various functions regarding the operation performed in the operating room. The acquired information from each source is used as necessary for execution of the medical device software.

The medical information processing system 300 may be implemented by a single device such as the operating room controller 107 and the external server 113 described above, or may be implemented by a plurality of these devices operating in cooperation.

The medical information processing system 300 includes a software list management unit 311, a source specification unit 312, an information acquisition unit 313, an analysis processing unit 314, a contraindication determination unit 315, and an activation processing unit 316.

The software list management unit 311 manages a software list that is a list of various types of software executed by the medical information processing system 300. The software list is managed for each patient. Contraindication information serving as a reference for determining whether or not activation of the software should be contraindicated is set in the software registered in the software list (registered software). For example, the contraindication information is set by decompressing the compressed and stored contraindication information when the medical device software is installed in the medical information processing system 300.

Furthermore, in the software list, the source (electronic device) to be referred to is set for each registered software. It is determined whether or not the activation of each registered software should be contraindicated on the basis of the acquired information from the source set in the software list.

In response to the activation request of the software from the user, the source specification unit 312 specifies the source (the source from which the acquired information is acquired) that should be referred to by the software requested to be activated (requested software). Specifically, the source specification unit 312 specifies the source that should be referred to by the requested software set in the requested software on the basis of the software list managed by the software list management unit 311. Information indicating the specified source is supplied to the information acquisition unit 313.

The information acquisition unit 313 acquires the acquired information (patient information and device information) from the source that should be referred to by the requested software on the basis of the information from the source specification unit 312. The patient information includes, for example, patient state information regarding a state of the patient, and the device information includes, for example, device state information regarding a state of the source (electronic device). The information acquisition unit 313 includes an electronic medical record acquisition unit 313a, an endoscopic image acquisition unit 313b, and an operating room image acquisition unit 313c.

In a case where the source indicated by the information from the source specification unit 312 is the electronic medical record system 210, the electronic medical record acquisition unit 313a acquires the electronic medical record including the patient information from the electronic medical record system 210 as the acquired information. In a case where the source indicated by the information from the source specification unit 312 is the endoscope camera 220, the endoscopic image acquisition unit 313b acquires the endoscopic image and an observation mode of the endoscope from the endoscope camera 220 as the acquired information. The observation mode of the endoscope includes a white light observation mode and an infrared light observation mode. In a case where the source indicated by the information from the source specification unit 312 is the operating room camera 230, the operating room image acquisition unit 313c acquires the operating room image from the operating room camera 230 as the acquired information.

The acquired information acquired in this manner is supplied to the analysis processing unit 314.

The analysis processing unit 314 generates analysis information by analyzing the acquired information from the information acquisition unit 313. The analysis processing unit 314 includes a patient information analysis unit 314a and an image analysis unit 314b.

In a case where the electronic medical record is supplied from the information acquisition unit 313, the patient information analysis unit 314a analyzes the patient information included in the electronic medical record, and outputs an analysis result thereof as the analysis information. In this case, the analysis information includes the state of the patient such as a medical history and an allergy history of the patient. In a case where the endoscopic image, the observation mode of the endoscope, and the operating room image are supplied from the information acquisition unit 313, the image analysis unit 314b analyzes these pieces of information, and outputs the analysis result thereof as the analysis information. In this case, the analysis information includes the state of the source (electronic device) and the like in addition to a surgical situation such as a surgical site of the patient, an operation of the operator, and an appliance.

The analysis information generated in this manner is supplied to the contraindication determination unit 315.

The contraindication determination unit 315 determines whether or not the activation of the requested software is contraindicated according to the contraindication information set in the requested software on the basis of the software list managed by the software list management unit 311. Specifically, the contraindication determination unit 315 determines whether or not the activation of the requested software is contraindicated according to whether or not the analysis information from the analysis processing unit 314 (eventually, the acquired information acquired by the information acquisition unit 313) corresponds to the contraindication information set in the requested software.

For example, the contraindication information includes, as the state of the patient to whom the requested software cannot be used, an allergy or a surgical situation of the patient. Furthermore, the contraindication information includes the observation mode (infrared light observation mode) of the endoscope and the like as the state of the source (electronic device) to which the requested software cannot be used.

A result of determination as to whether or not the activation of the requested software is contraindicated is supplied to the activation processing unit 316.

The activation processing unit 316 controls the activation of the requested software on the basis of the determination result from the contraindication determination unit 315. Specifically, in a case where the determination result from the contraindication determination unit 315 indicates that it is not determined that the activation of the requested software should be contraindicated, the activation processing unit 316 activates the requested software. In contrast, in a case where the determination result from the contraindication determination unit 315 indicates that it is determined that the activation of the requested software should be contraindicated, the activation processing unit 316 stops activating the requested software.

### (Flow of Software Activation Determination Processing)

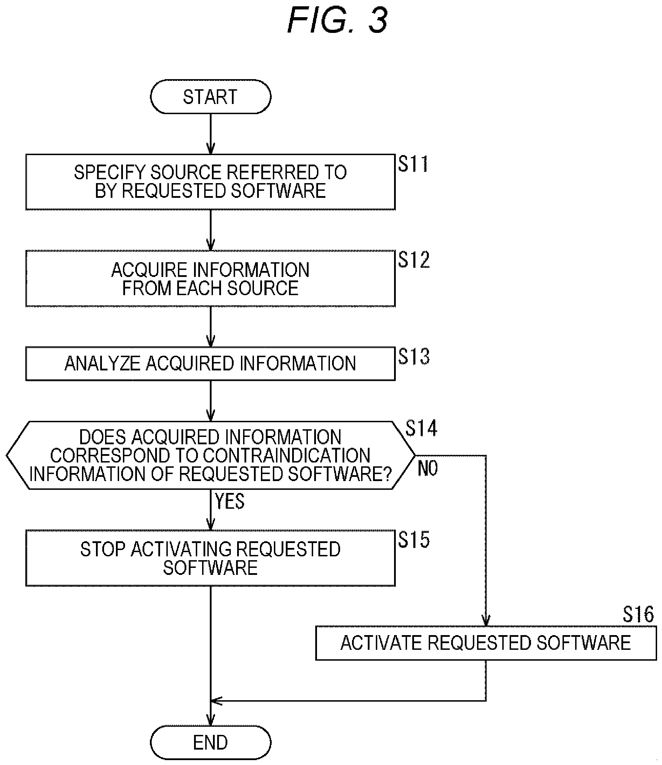

Next, a flow of software activation determination processing performed by the medical information processing system 300 in Fig. 2 will be described with reference to a flowchart in Fig. 3. The processing in Fig. 3 is started with a software activation request from the user as a trigger.

At step 511, the source specification unit 312 specifies the source referred to by the requested software in response to the activation request.

At step S12, the information acquisition unit 313 acquires the acquired information from each source specified by the source specification unit 312.

At step S13, the analysis processing unit 314 generates the analysis information by analyzing the acquired information acquired by the information acquisition unit 313.

At step S14, the contraindication determination unit 315 determines whether or not the analysis information generated by the analysis processing unit 314 corresponds to the contraindication information set in the requested software on the basis of the software list of the software list management unit 311.

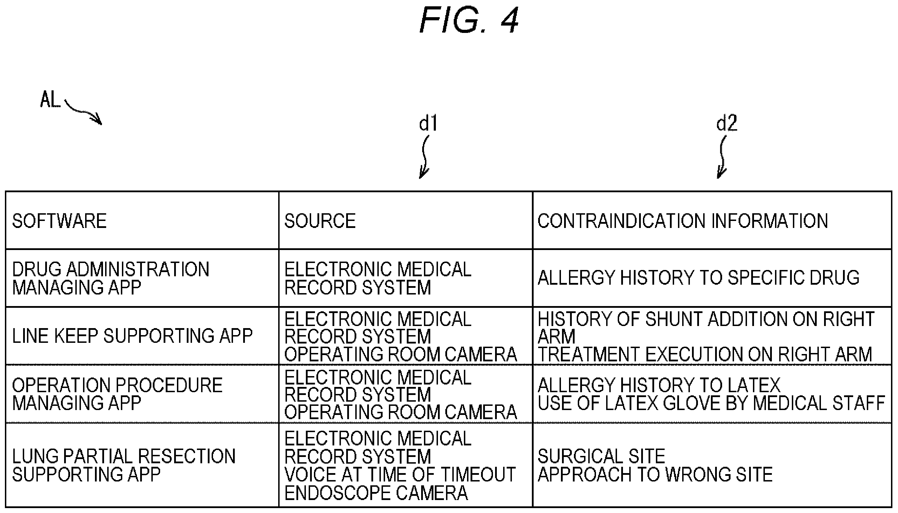

Fig. 4 is a diagram illustrating an example of the software list.

In a software list AL illustrated in Fig. 4, a source d1 and contraindication information d2 are set for each registered software.

For example, in a drug administration managing app (application), which is software that manages a drug to be administered to the patient, "electronic medical record system" is set as the source d1. Furthermore, in this app, "allergy history to specific drug" is set as the contraindication information d2.

In this case, it is determined whether or not the analysis result of the electronic medical record from the electronic medical record system 210 includes "allergy history to specific drug".

Furthermore, "electronic medical record system" and "operating room camera" are set as the source d1 in a line keep supporting app, which is software that supports blood vessel securement for drip infusion and injection. Furthermore, in the app, "history of shunt addition on right arm" and "treatment execution on right arm" are set as the contraindication information d2.

In this case, it is determined whether or not the analysis result of the electronic medical record from the electronic medical record system 210 includes "history of shunt addition on right arm". Furthermore, it is determined whether or not the analysis result of the operating room image from the operating room camera 230 includes "record of treatment execution on right arm".

Moreover, "electronic medical record system" and "operating room camera" are set as the source d1 in an operation procedure managing app, which is software that guides the operator in the procedure of operation technique. Furthermore, "allergy history to latex" and "use of latex gloves by medical staff" are set as the contraindication information d2 in the app.

In this case, it is determined whether or not the analysis result of the electronic medical record from the electronic medical record system 210 includes "allergy history to latex". Furthermore, it is determined whether or not the analysis result of the operating room image from the operating room camera 230 includes "use of latex gloves by operator".

Furthermore, "electronic medical record system", "voice at the time of timeout", and "endoscope camera" are set as the source d1 in a lung partial resection supporting app, which is software that supports lung partial resection. The timeout refers to a confirmation of the patient name, the surgical method including an operation method and a surgical site (right and left) and the like before starting the operation. Therefore, here, "voice at the time of timeout" as the source d1 indicates a microphone and the like to which voice of the operator can be input before starting the operation. Furthermore, "surgical site" and "approach to wrong site" are set as the contraindication information d2 in the app.

In this case, on the basis of the analysis result of the electronic medical record from the electronic medical record system 210 and "surgical site" included in the analysis result of the voice from the microphone, it is determined whether or not the analysis result of the endoscopic image from the endoscope camera 220 includes "approach to wrong site (different from the surgical site)".

In this manner, the contraindication determination unit 515 determines whether or not the analysis information corresponds to the contraindication information set in the requested software on the basis of the software list.

Returning to the flowchart in Fig. 3, in a case where it is determined at step S14 that the analysis information corresponds to the contraindication information set in the requested software, the processing proceeds to step S15, and the activation processing unit 316 stops activating the requested software.

In contrast, in a case where it is determined at step S14 that the analysis information does not correspond to the contraindication information set in the requested software, the processing proceeds to step S16, and the activation processing unit 316 activates the requested software.

According to the above-described processing, since the system checks whether or not the activation of the medical device software should be prohibited due to a possible adverse effect by a patient-specific factor, it is possible to prevent inappropriate use of the medical device software. As a result, it is possible to avoid the patient from being damaged invasively by the activation of the medical device software that should be contraindicated.

### <4. Second Embodiment>

In the operating room system 100 according to the above-described embodiment, there may be a case where, even in a state in which activation of certain software is determined to be contraindicated, the software needs to be activated according to judgment of a doctor.

Therefore, a configuration of the operating room system 100 capable of activating the software the activation of which is determined to be contraindicated by the judgment of the doctor (user) will be hereinafter described.

### (Functional Configuration Example of Operating Room System)

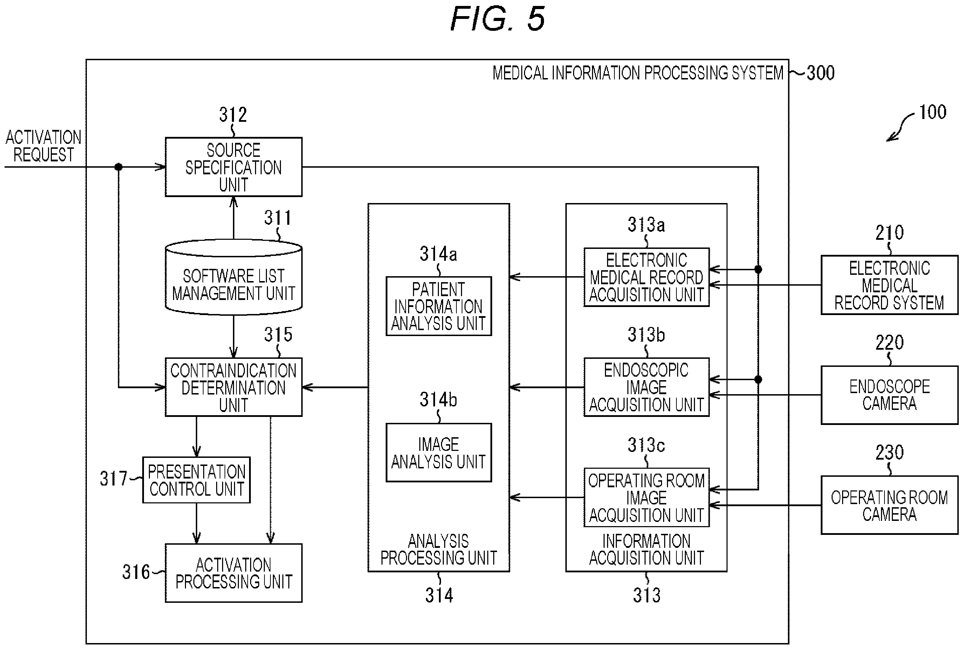

Fig. 5 is a diagram illustrating a functional configuration example of an operating room system 100 according to a second embodiment of the present disclosure.

The operating room system 100 in Fig. 5 is different from the operating room system 100 in Fig. 2 in that a presentation control unit 317 is newly provided in a medical information processing system 300.

The presentation control unit 317 presents selection information for allowing a user to select necessity of activation of requested software on the basis of a determination result from a contraindication determination unit 315. Specifically, in a case where the determination result from the contraindication determination unit 315 indicates that it is determined that the activation of the requested software should be contraindicated, the presentation control unit 317 presents the selection information. The selection information is displayed on, for example, a centralized operation panel 111 or any one of display devices 103A to 103D.

In a case where the selection information is presented, an activation processing unit 316 controls the activation of the requested software according to a selection result for the selection information.

### (Flow of Software Activation Determination Processing)

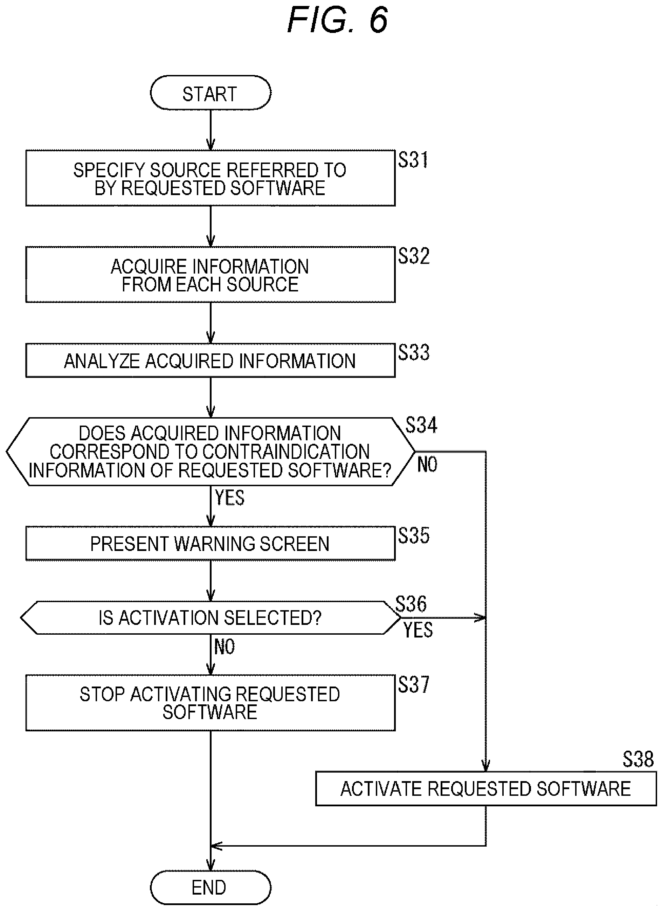

Next, a flow of software activation determination processing performed by the medical information processing system 300 in Fig. 5 will be described with reference to a flowchart in Fig. 6. The processing in Fig. 6 is also started with a software activation request from the user as a trigger.

Note that, processing at steps S31 to S34 in the flowchart in Fig. 6 is similar to the processing at steps S11 to S14 in the flowchart in Fig. 3, so that the description thereof will be omitted.

That is, in a case where it is determined at step S34 that the analysis information corresponds to contraindication information set in the requested software, the processing proceeds to step S35, and the presentation control unit 317 presents a warning screen including the selection information.

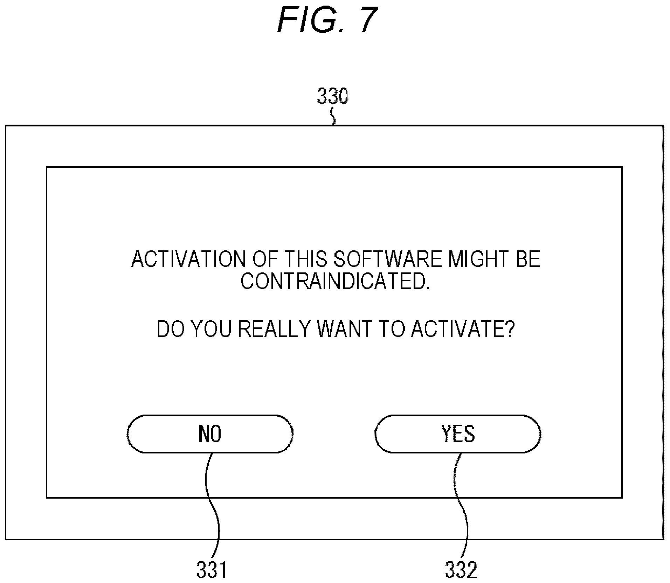

Fig. 7 is a diagram illustrating an example of the warning screen.

A warning screen 330 illustrated in Fig. 7 displays a NO button 331 and a YES button 332 as the above-described selection information in addition to a warning message such as "Activation of this software might be contraindicated. Do you really want to activate?"

The NO button 331 is a button selected to stop the activation of the requested software determined to be contraindicated. In contrast, the YES button 332 is a button selected to activate the requested software determined to be contraindicated.

Then, at step S36, the presentation control unit 317 determines whether or not the activation of the requested software is selected.

In a case where it is determined at step S36 that the activation of the requested software is not selected, that is, in a case where the NO button 331 is selected on the warning screen 330, the processing proceeds to step S37, and the activation processing unit 316 stops activating the requested software.

In contrast, in a case where it is determined at step S36 that the activation of the requested software is selected, that is, in a case where the YES button 332 is selected on the warning screen 330, the processing proceeds to step S38, and the activation processing unit 316 activates the requested software.

Note that, in a case where it is determined at step S34 that the analysis information corresponds to the contraindication information set in the requested software, the processing proceeds to step S38, and the activation processing unit 316 activates the requested software.

According to the above-described processing, even in a state in which the activation of the requested software is determined to be contraindicated, it is possible to decide whether to activate the software or stop activating the software by the judgment of the user.

### <5. Third Embodiment>

In the operating room system 100 according to the above-described embodiment, in a case where the medical information processing system 300 cannot be connected to an external device such as the electronic medical record system 210, the endoscope camera 220, the operating room camera 230 and the like, there may be a case where the requested software needs to be activated according to the judgment of the doctor.

Therefore, an operation of the operating room system 100 capable of activating requested software by the judgment of the doctor (user) in an emergency such as a case where the medical information processing system 300 cannot be connected to an external device will be hereinafter described.

### (Flow of Software Activation Determination Processing)

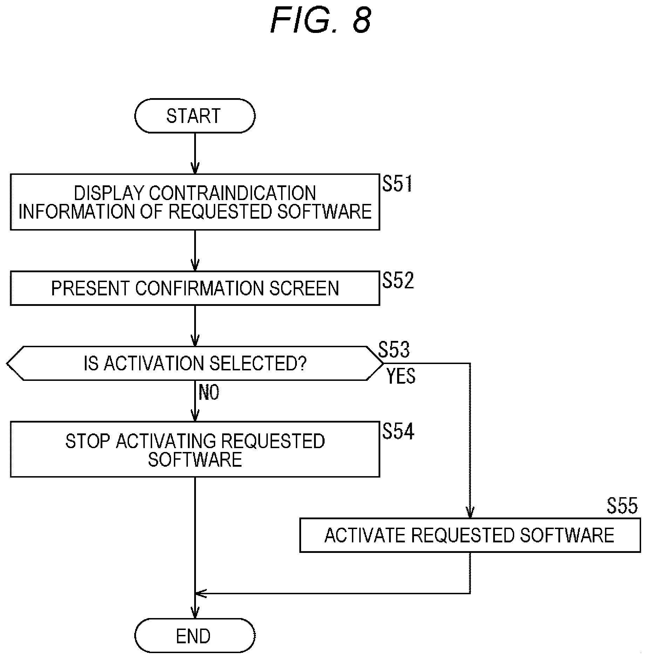

A flow of software activation determination processing in an emergency mode performed by the medical information processing system 300 in Fig. 5 will be described with reference to a flowchart in Fig. 8. Processing in Fig. 8 is started when the contraindication determination unit 315 acquires the contraindication information set in the requested software on the basis of the software list of the software list management unit 311 with the activation request of the software from the user as a trigger.

At step S51, the presentation control unit 317 displays the contraindication information of the requested software acquired by the contraindication determination unit 315.

At step S52, the presentation control unit 317 presents a confirmation screen including selection information. The confirmation screen is a screen on which a NO button and a YES button as the selection information described above are displayed, similarly to the warning screen described with reference to Fig. 7.

At step S53, the presentation control unit 317 determines whether or not the activation of the requested software is selected.

In a case where it is determined at step S54 that the activation of the requested software is not selected, that is, in a case where the NO button is selected on the confirmation screen, the processing proceeds to step S55, and the activation processing unit 316 stops activating the requested software.

In contrast, in a case where it is determined at step S53 that the activation of the requested software is selected, that is, in a case where the YES button is selected on the confirmation screen, the processing proceeds to step S55, and the activation processing unit 316 activates the requested software.

According to the above-described processing, in an emergency such as a case where the medical information processing system 300 cannot be connected to the external device, the user himself/herself can decide whether to activate the requested software or stop activating the software after confirmation of the state of the patient and the situation.

### <6. Fourth Embodiment>

In the operating room system 100 of the above-described embodiment, it is determined whether or not the activation of the requested software should be contraindicated by referring to the contraindication information set in the software list. There is no limitation, and contraindication candidate information that is a candidate for the contraindication information may be generated by activated software activated for a specific patient.

In this case, activation availability information indicating activation availability is set for the software registered in the software list of the patient on the basis of the generated contraindication candidate information.

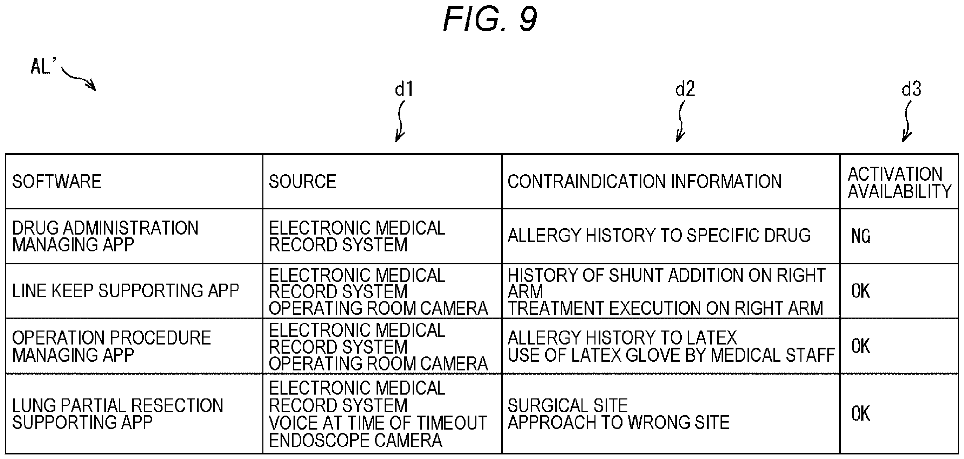

Fig. 9 is a diagram illustrating an example of a software list in which the activation availability information is set.

In a software list AL' illustrated in Fig. 9, activation availability information d3 for each registered software is set in addition to a source d1 and contraindication information d2 set for each registered software similar to the software list AL in Fig. 4.

As the activation availability information d3, either "OK" that does not contraindicate activation of the software for the patient or "NG" that contraindicates the activation of the software is set. In the example in Fig. 9, NG is set as the activation availability information d3 in a drug administration managing app, and OK is set as the activation availability information d3 in each of a line keep supporting app, an operation procedure managing app, and a lung partial resection supporting app.

The activation availability information d3 in the software list AL' is updated on the basis of the contraindication candidate information generated by the activated software activated for the target patient.

Therefore, a configuration of the operating room system 100 that updates the activation availability information in the software list on the basis of the contraindication candidate information generated by the activated software will be hereinafter described.

### (Functional Configuration Example of Operating Room System)

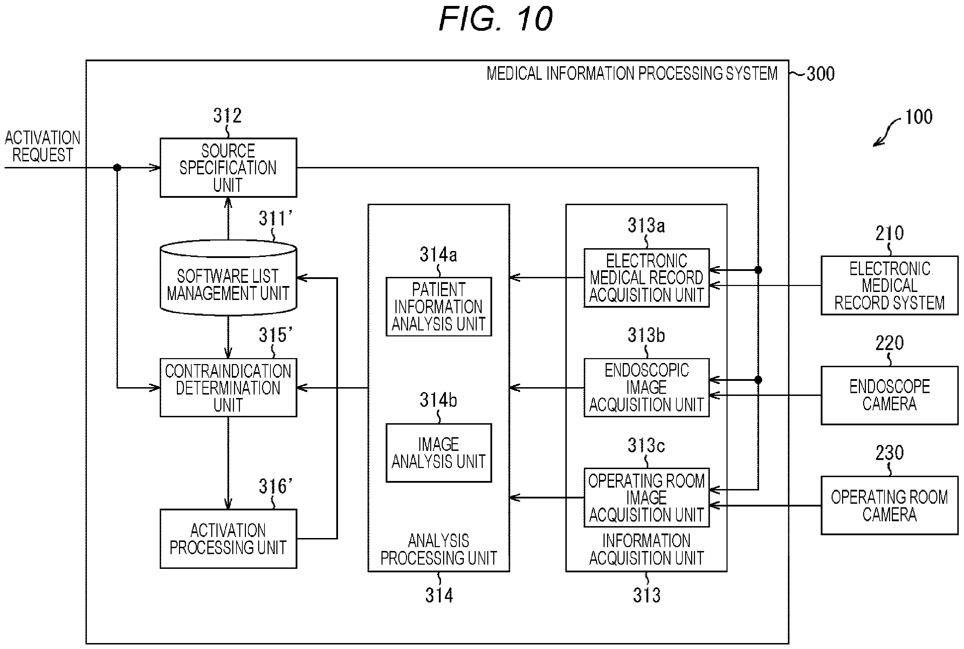

Fig. 10 is a diagram illustrating a functional configuration example of an operating room system 100 according to a fourth embodiment of the present disclosure.

The operating room system 100 in Fig. 10 is different from the operating room system 100 in Fig. 2 in that a software list management unit 311', a contraindication determination unit 315', and an activation processing unit 316' are provided in place of the software list management unit 311, the contraindication determination unit 315, and the activation processing unit 316 in the medical information processing system 300.

The software list management unit 311' manages the software list for each patient in which the activation availability information is set for each registered software as described with reference to Fig. 9. In the software list in an initial state, "OK" is set as the activation availability information of each registered software.

The contraindication determination unit 315' has a function similar to that of the contraindication determination unit 315 in Fig. 2, and determines whether or not the requested software should be contraindicated according to the activation availability information set in the requested software.

The activation processing unit 316' supplies, in a case where the contraindication candidate information is generated by the activated software activated for the target patient, the generated contraindication candidate information to the software list management unit 311'. The software list management unit 311' updates the activation availability information in the software list on the basis of the contraindication candidate information supplied from the activation processing unit 316' .

### (Flow of List Update Processing)

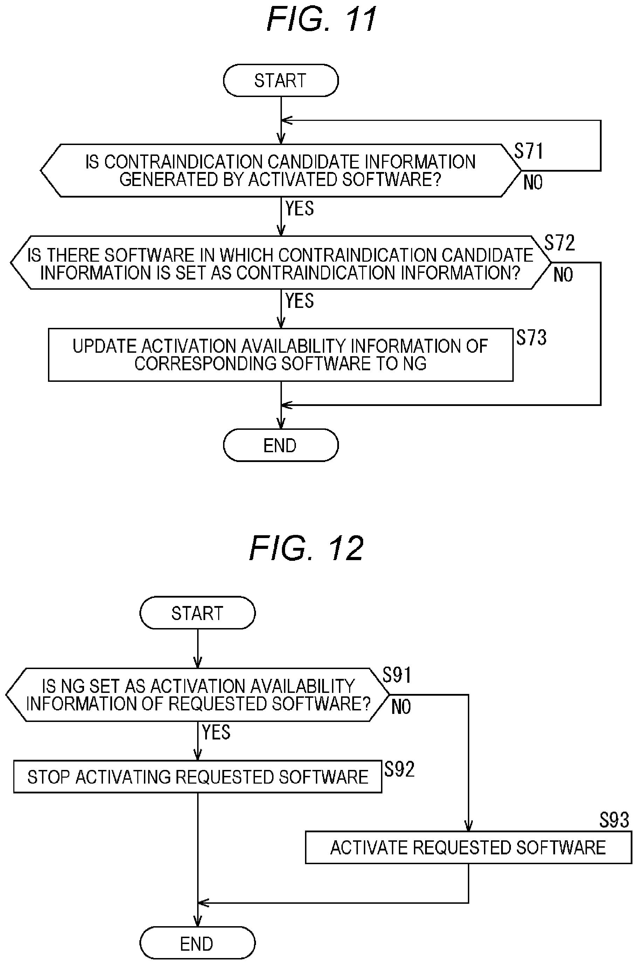

Next, a flow of list update processing performed by the medical information processing system 300 in Fig. 10 will be described with reference to a flowchart in Fig. 11. The processing in Fig. 11 is started in a state in which the software is activated for a predetermined patient.

At step S71, the activation processing unit 316' determines whether or not the contraindication candidate information is generated by the activated software activated for the target patient. Step S71 is repeated until it is determined that the contraindication candidate information is generated. When it is determined that the contraindication candidate information is generated, the processing proceeds to step S72.

At step S72, the software list management unit 311' determines whether or not there is software in which the contraindication candidate information generated by the activated software is set as the contraindication information in the software list of the target patient. In a case where it is determined that there is the software in which the generated contraindication candidate information is set as the contraindication information, the processing proceeds to step S73.

At step S73, the software list management unit 311' updates the activation availability information of the corresponding software (software in which the contraindication candidate information is set as the contraindication information) from OK to NG in the software list of the target patient.

Note that, in a case where it is determined that there is no software in which the generated contraindication candidate information is set as the contraindication information at step S72, step S73 is skipped.

In the above-described manner, the activation availability information of the software list is updated on the basis of the contraindication candidate information generated by the activated software.

### (Flow of Software Activation Determination Processing)

Next, a flow of software activation determination processing performed by the medical information processing system 300 in Fig. 10 will be described with reference to a flowchart in Fig. 12. The processing in Fig. 12 is also started with a software activation request from the user as a trigger.

At step S91, the contraindication determination unit 315' determines whether or not NG is set in the activation availability information of the requested software in the software list of the target patient managed by the software list management unit 311'.

In a case where it is determined at step S91 that NG is set in the activation availability information of the requested software, the processing proceeds to step S92, and the activation processing unit 316' stops activating the requested software.

In contrast, in a case where it is determined at step S91 that NG is not set in the activation availability information of the requested software, the processing proceeds to step S93, and the activation processing unit 316' activates the requested software.

According to the above-described processing also, since the system checks whether or not the activation of the medical device software should be prohibited due to a possible adverse effect by a patient-specific factor, it is possible to prevent inappropriate use of the medical device software. As a result, it is possible to avoid the patient from being damaged invasively by the activation of the medical device software that should be contraindicated.

In the above-described processing, in a case where NG is set in the activation availability information of the requested software, the activation availability of the software may be decided by the judgment of the user by displaying the warning screen similarly to the processing in Fig. 6.

Note that, in the software list, there may be software in which NG is permanently set as the activation availability information for the patient and software in which NG is temporarily set as the activation availability information as for the software in which NG is set once in the activation availability information.

For example, as for software related to a state or a situation continuously continuing once treated or generated, such as generation of a shunt or allergy, NG is permanently set as the activation availability information for the patient. Furthermore, NG is temporarily set as the activation availability information for the patient as for the software related to the temporary state or situation, such as a drug expected to have an effect only for a specific period.

In this case, in the medical information processing system 300, the source is periodically referred to, and it is determined whether the activation availability information of the corresponding software is OK or NG.

### <7. Fifth Embodiment>

In the operating room system 100 according to the above-described embodiment, after it is determined that activation of certain software is not contraindicated, there may be a case where the software is updated or the state of the patient is updated while the software is activated.

Therefore, a configuration of an operating room system 100 that determines, in a case where contraindication information of activated software and patient information are updated, activation availability of the software is hereinafter described.

### (Functional Configuration Example of Operating Room System)

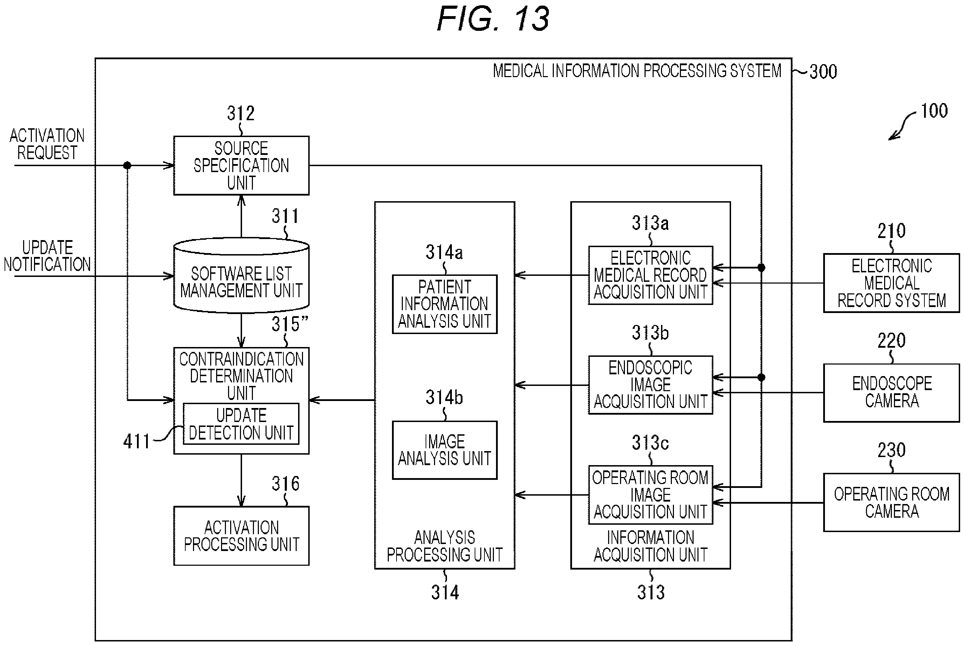

Fig. 13 is a diagram illustrating a functional configuration example of an operating room system 100 according to a fifth embodiment of the present disclosure.

The operating room system 100 in Fig. 13 is different from the operating room system 100 in Fig. 2 in that a contraindication determination unit 315'' is provided in place of the contraindication determination unit 315 in the medical information processing system 300.

The contraindication determination unit 315'' has a function similar to that of the contraindication determination unit 315 in Fig. 2, and further includes an update detection unit 411.

The update detection unit 411 detects the contraindication information of the registered software in the software list managed by the software list management unit 311 and the update of the patient information acquired by the information acquisition unit 313.

For example, the update detection unit 411 detects the update of the contraindication information of the registered software according to update notification of the medical device software from the external device to the software list management unit 311. Furthermore, the update detection unit 411 detects the update of the patient information on the basis of the state of the patient output as the analysis information obtained by analyzing a patient image obtained by imaging the patient by the analysis processing unit 314.

Then, the contraindication determination unit 315'' determines whether or not activation of the activated requested software should be contraindicated on the basis of updated contents of the contraindication information and the patient information.

### (Flow of Software Activation Determination Processing)

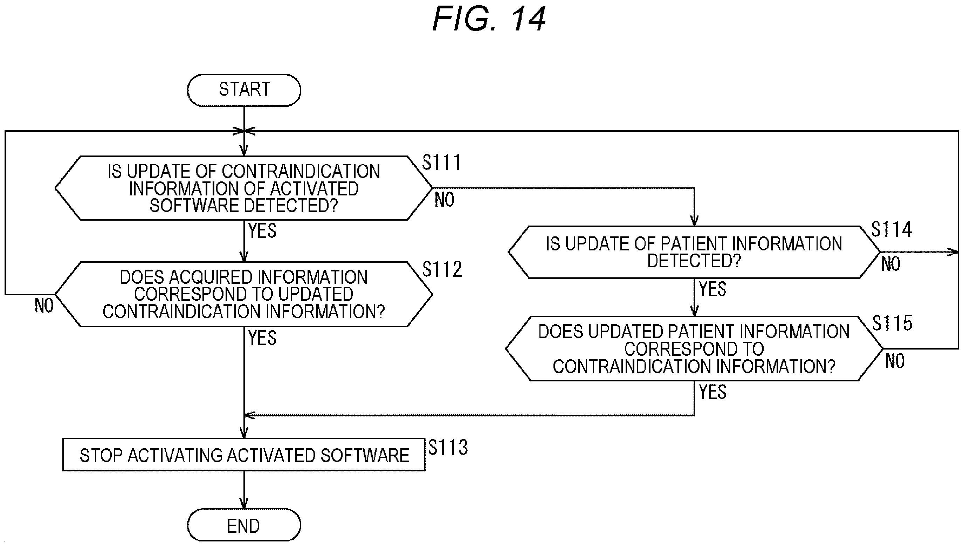

A flow of software activation determination processing performed by the medical information processing system 300 in Fig. 13 will be described with reference to a flowchart in Fig. 14. The processing in Fig. 14 is executed, for example, after the activated software is activated.

At step S111, the update detection unit 411 determines whether or not the update of the contraindication information of the activated software is detected.

In a case where it is determined at step Sill that the update of the contraindication information of the activated software is detected, the processing proceeds to step S112.

At step S112, the contraindication determination unit 315'' determines whether or not the patient information or the device information, which is the acquired information acquired by the information acquisition unit 313, corresponds to the updated contraindication information.

In a case where it is determined at step S112 that the acquired information corresponds to the updated contraindication information, the processing proceeds to step S113, and the activation processing unit 316' stops activating the activated software.

In contrast, in a case where it is determined at step S112 that the acquired information does not correspond to the updated contraindication information, the processing returns to step S111, and the subsequent processing is repeated in a state in which the activation of the activated software is continued.

In a case where it is determined at step Sill that the update of the contraindication information of the activated software is not detected, the processing proceeds to step S114, and the update detection unit 411 determines whether or not the update of the patient information is detected.

In a case where it is determined that the update of the patient information is detected at step S114, the processing proceeds to step S115.

At step S115, the contraindication determination unit 315'' determines whether or not the updated patient information corresponds to the contraindication information.

In a case where it is determined at step S115 that the updated patient information corresponds to the contraindication information, the processing proceeds to step S113, and the activation processing unit 316' stops activating the activated software.

In contrast, in a case where it is determined at step S115 that the updated patient information does not correspond to the contraindication information, the processing returns to step S111, and the subsequent processing is repeated in a state in which the activation of the activated software is continued.

Furthermore, in a case where it is determined at step S114 that the update of the patient information is not detected, that is, in a case where the update of neither the contraindication information of the activated software nor the patient information is detected, the processing returns to step S111, and the subsequent processing is repeated in a state in which the activation of the activated software is continued.

According to the above-described processing, since the system checks whether or not the activation of the activated medical device software should be stopped due to a possible adverse effect by update of the contraindication information and the patient information, it is possible to prevent inappropriate use of the medical device software. As a result, it is possible to avoid the patient from being damaged invasively by the activation of the medical device software that should be contraindicated.

### <8. Sixth Embodiment>

In the operating room system 100 of the above-described embodiment, as for the software registered in the software list, either first contraindication information indicating that the software cannot be used or second contraindication information indicating that the software can be used with a condition may be set as the contraindication information.

For example, the second contraindication information includes, as the condition, a state of a patient or a state of a device to whom (which) the software cannot be used.

In this case, when the second contraindication information is set in the activated software, continuation or stop of the activation of the software is determined by a change in patient information or device information acquired as acquired information.

### (Flow of Software Activation Determination Processing)

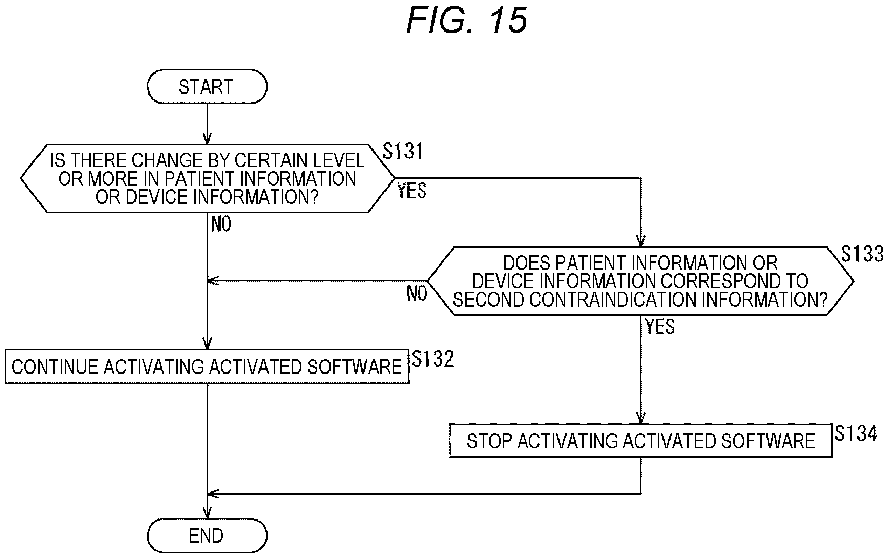

A flow of software activation determination processing in a case where the second contraindication information is set in the activated software in the medical information processing system 300 in Fig. 13, for example, is described with reference to the flowchart in Fig. 15. The processing in Fig. 15 is executed every constant period of time (periodically), for example, after the activated software is activated.

At step S131, the analysis processing unit 314 analyzes the patient information or the device information as the acquired information to determine whether or not there is a change by a certain level or more in the patient information or the device information. The change in patient information and device information herein mentioned is a change in a direction in which the state of the patient and the state of the device do not satisfy the condition included in the second contraindication information.

In a case where it is determined at step S131 that the patient information or the device information does not change by a certain level or more, the processing proceeds to step S132, and the activation processing unit 316 continues activating the activated software.

In contrast, in a case where it is determined at step S131 that there is a change by a certain level or more in the patient information or the device information, the processing proceeds to step S133.

At step S133, the contraindication determination unit 315'' determines whether or not the patient information or the device information analyzed by the analysis processing unit 314 corresponds to the second contraindication information of the activated software.

In a case where it is determined at step S133 that the patient information or the device information does not correspond to the second contraindication information of the activated software, the processing proceeds to step S132, and the activation processing unit 316 continues activating the activated software.

In contrast, in a case where it is determined at step S133 that the patient information or the device information corresponds to the second contraindication information of the activated software, the processing proceeds to step S134, and the activation processing unit 316 stops activating the activated software.

According to the above-described processing, since the system checks whether or not the activation of the activated medical device software should be stopped due to a possible adverse effect by a change in patient state and device state, it is possible to prevent inappropriate use of the medical device software. As a result, it is possible to avoid the patient from being damaged invasively by the activation of the medical device software that should be contraindicated.

### <9. Configuration Example of Computer>

The series of processing described above can be executed by hardware and also can be executed by software. In a case where the series of processing is executed by software, a program included in the software is installed from a program recording medium to a computer incorporated in dedicated hardware, a general-purpose personal computer and the like.

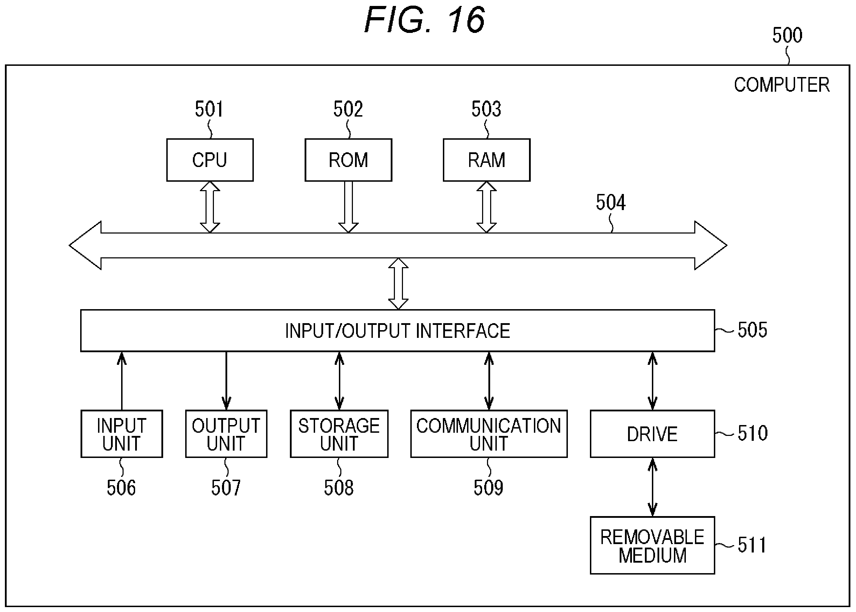

Fig. 16 is a block diagram illustrating a configuration example of hardware of a computer that executes the above-described series of processing by a program.

The medical information processing system 300 to which the technology according to the present disclosure can be applied may be implemented by the computer having the configuration illustrated in Fig. 16.

A CPU 501, a read only memory (ROM) 502, and a random access memory (RAM) 503 are mutually connected over a bus 504.

An input/output interface 505 is further connected to the bus 504. An input unit 506 including a keyboard, a mouse and the like, and an output unit 507 including a display, a speaker and the like are connected to the input/output interface 505. Furthermore, a storage unit 508 including a hard disk, a nonvolatile memory and the like, a communication unit 509 including a network interface and the like, and a drive 510 that drives a removable medium 511 are connected to the input/output interface 505.

In the computer configured as described above, for example, the CPU 501 loads a program stored in the storage unit 508 into the RAM 503 via the input/output interface 505 and the bus 504 and executes the program to perform the above-described series of processing.

For example, the program executed by the CPU 501 is recorded in the removable medium 511, or provided via a wired or wireless transmission medium such as a local area network, the Internet, or digital broadcasting, and then installed in the storage unit 508.

Note that, the program executed by the computer may be a program in which processing is performed in time series in the order described herein, or may be a program in which processing is performed in parallel or at necessary timing such as when a call is made and the like.

Embodiments of the present disclosure are not limited to the above-described embodiments, and various modifications can be made in a range without departing from the gist of the present disclosure.

Furthermore, the effects described herein are merely examples and are not limited, and there may be other effects.

Moreover, the present disclosure may have the following configurations.
(1) A medical information processing system including:
   a list management unit that manages a software list in which contraindication information is set for each registered software;
   an information acquisition unit that acquires patient information regarding a patient to whom requested software requested to be activated is used and device information regarding an electronic device used for the patient; and
   a contraindication determination unit that determines whether or not activation of the requested software should be contraindicated according to whether or not the patient information or the device information corresponds to the contraindication information set in the requested software in the software list.
(2) The medical information processing system according to (1), further including:
   a detection unit that detects update of the contraindication information of the registered software or the patient information, in which
   the contraindication determination unit determines whether or not the activation of the requested software should be contraindicated on the basis of updated contents of the contraindication information or the patient information.
(3) The medical information processing system according to (2), in which
   in a case where the update of the contraindication information is detected, the contraindication determination unit determines whether or not the patient information or the device information corresponds to the updated contraindication information.
(4) The medical information processing system according to (2), in which
   in a case where the update of the patient information is detected, the contraindication determination unit determines whether or not the updated patient information corresponds to the contraindication information.
(5) The medical information processing system according to any one of (2) to (4), in which
   the contraindication information includes a state of the patient to whom the registered software cannot be used, and
   the information acquisition unit acquires patient state information regarding the state of the patient as the patient information.
(6) The medical information processing system according to (5), in which
   the state of the patient includes an allergy or a surgical situation.
(7) The medical information processing system according to (6), further including:
   an analysis processing unit that outputs the state of the patient as analysis information obtained by analyzing a patient image obtained by imaging the patient, and
   the detection unit detects the update of the patient information on the basis of the analysis information.
(8) The medical information processing system according to any one of (2) to (4), in which
   the contraindication information includes a state of the electronic device to which the registered software cannot be used, and
   the information acquisition unit acquires device state information regarding the state of the electronic device as the device information.
(9) The medical information processing system according to (8), in which
   the state of the electronic device includes an observation mode of an endoscope.
(10) The medical information processing system according to any one of (1) to (9), in which
   the contraindication information includes first contraindication information indicating that the registered software cannot be used and second contraindication information indicating that the registered software can be used with a condition, and
   the contraindication determination unit determines whether or not the patient information or the device information corresponds to the second contraindication information.
(11) The medical information processing system according to (10), in which
   in a case where a change by a certain level or more occurs in the patient information or the device information, the contraindication determination unit determines whether or not the patient information or the device information corresponds to the second contraindication information.
(12) The medical information processing system according to any one of (1) to (11), further including:
   an activation processing unit that executes processing related to the activation of the requested software, in which
   the activation processing unit stops activating the requested software in a case where it is determined that the activation of the requested software should be contraindicated.
(13) The medical information processing system according to (12), further including:
   a presentation control unit that presents selection information for selection of necessity of the activation of the requested software in a case where it is determined that the activation of the requested software should be contraindicated, in which
   the activation processing unit activates the requested software in a case where it is selected that the activation of the requested software is necessary for the selection information.
(14) A determining method including:
   by a medical information processing system
   managing a software list in which contraindication information is set for each registered software;
   acquiring patient information regarding a patient to whom requested software requested to be activated is used and device information regarding an electronic device used for the patient; and
   determining whether or not activation of the requested software should be contraindicated according to whether or not the patient information or the device information corresponds to the contraindication information set in the requested software in the software list.
(15) A program for allowing a computer to execute processing of:
   managing a software list in which contraindication information is set for each registered software;
   acquiring patient information regarding a patient to whom requested software requested to be activated is used and device information regarding an electronic device used for the patient; and
   determining whether or not activation of the requested software should be contraindicated according to whether or not the patient information or the device information corresponds to the contraindication information set in the requested software in the software list.

### REFERENCE SIGNS LIST

- 100: Operating room system
- 300: Medical information processing system
- 311, 311': Software list management unit
- 312: Source determination unit
- 313: Information acquisition unit
- 314: Analysis processing unit
- 315, 315', 315'': Contraindication determination unit
- 316, 316': Activation processing unit
- 317: Presentation control unit
- 411: Update detection unit

## Claims

1. A medical information processing system comprising:
a list management unit that manages a software list in which contraindication information is set for each registered software;
an information acquisition unit that acquires patient information regarding a patient to whom requested software requested to be activated is used and device information regarding an electronic device used for the patient; and
a contraindication determination unit that determines whether or not activation of the requested software should be contraindicated according to whether or not the patient information or the device information corresponds to the contraindication information set in the requested software in the software list.

2. The medical information processing system according to claim 1, further comprising:
a detection unit that detects update of the contraindication information of the registered software or the patient information, wherein
the contraindication determination unit determines whether or not the activation of the requested software should be contraindicated on a basis of updated contents of the contraindication information or the patient information.

3. The medical information processing system according to claim 2, wherein
in a case where the update of the contraindication information is detected, the contraindication determination unit determines whether or not the patient information or the device information corresponds to the updated contraindication information.

4. The medical information processing system according to claim 2, wherein
in a case where the update of the patient information is detected, the contraindication determination unit determines whether or not the updated patient information corresponds to the contraindication information.

5. The medical information processing system according to claim 2, wherein
the contraindication information includes a state of the patient to whom the registered software cannot be used, and
the information acquisition unit acquires patient state information regarding the state of the patient as the patient information.

6. The medical information processing system according to claim 5, wherein
the state of the patient includes an allergy or a surgical situation.

7. The medical information processing system according to claim 6, further comprising:
an analysis processing unit that outputs the state of the patient as analysis information obtained by analyzing a patient image obtained by imaging the patient, and
the detection unit detects the update of the patient information on a basis of the analysis information.

8. The medical information processing system according to claim 2, wherein
the contraindication information includes a state of the electronic device to which the registered software cannot be used, and
the information acquisition unit acquires device state information regarding the state of the electronic device as the device information.

9. The medical information processing system according to claim 8, wherein
the state of the electronic device includes an observation mode of an endoscope.

10. The medical information processing system according to claim 1, wherein
the contraindication information includes first contraindication information indicating that the registered software cannot be used and second contraindication information indicating that the registered software can be used with a condition, and
the contraindication determination unit determines whether or not the patient information or the device information corresponds to the second contraindication information.

11. The medical information processing system according to claim 10, wherein
in a case where a change by a certain level or more occurs in the patient information or the device information, the contraindication determination unit determines whether or not the patient information or the device information corresponds to the second contraindication information.

12. The medical information processing system according to claim 1, further comprising:
an activation processing unit that executes processing related to the activation of the requested software, wherein
the activation processing unit stops activating the requested software in a case where it is determined that the activation of the requested software should be contraindicated.

13. The medical information processing system according to claim 12, further comprising:
a presentation control unit that presents selection information for selection of necessity of the activation of the requested software in a case where it is determined that the activation of the requested software should be contraindicated, wherein
the activation processing unit activates the requested software in a case where it is selected that the activation of the requested software is necessary for the selection information.

14. A determining method comprising:
by a medical information processing system
managing a software list in which contraindication information is set for each registered software;
acquiring patient information regarding a patient to whom requested software requested to be activated is used and device information regarding an electronic device used for the patient; and
determining whether or not activation of the requested software should be contraindicated according to whether or not the patient information or the device information corresponds to the contraindication information set in the requested software in the software list.

15. A program for allowing a computer to execute processing of:
managing a software list in which contraindication information is set for each registered software;
acquiring patient information regarding a patient to whom requested software requested to be activated is used and device information regarding an electronic device used for the patient; and
determining whether or not activation of the requested software should be contraindicated according to whether or not the patient information or the device information corresponds to the contraindication information set in the requested software in the software list.
